# EUROPEAN PATENT SPECIFICATION

(11) **EP 3 314 996 B1**
(45) Date of publication and mention of the grant of the patent: **17.06.2020**
(21) Application number: 17197916.4
(22) Date of filing: 24.10.2017
(51) Int. Cl.: A01B 69/04, A01F 15/08, A01D 91/04, A01D 78/00, G01N 33/24

(54) **A CROP MANAGEMENT SYSTEM FOR PROCESSING CROP MATERIAL**
ERNTEGUTMANAGEMENTSYSTEM ZUR VERARBEITUNG VON ERNTEGUT
SYSTÈME DE GESTION DE RÉCOLTE POUR LE TRAITEMENT DE MATÉRIAU RÉCOLTÉ

(30) Priority: 28.10.2016 US 201662414521 P; 21.08.2017 US 201715682312
(43) Date of publication of application: 02.05.2018
(73) Proprietor: Deere & Company, Moline, IL 61265 (US)
(72) Inventor: Murray, Cole L, 68163 Mannheim (DE); Townsell, Joe JR, 68163 Mannheim (DE)
(74) Representative: Reichert, Christian

(56) References cited:
- WO-A1-2010/003421
- WO-A1-2016/070195
- DE-A1-102011 100 054
- US-A1- 2012 109 614

## Description

The disclosure relates to a system for collecting and using data regarding the moisture content of crop or crop material during the haymaking process.

### BACKGROUND

Hay or bale creation includes a number of individual steps, such as cutting, tedding, raking, baling, and chemical application needed to prepare, collect, and bale the crop material for later use. In many instances, the crop material being baled is subsequently used for feed, being fed to farm animals and the like for nutrition and sustenance. As such, the bale creation process attempts to maximize the level of nutrition contained in the crop material so as to increase the level of nutrition contained in each bale. Furthermore, the bale creation process assures the crop material is able to dry out before it is baled to avoid mold and spoilage. Together, these two goals are generally at odds with one another such that assisting one is typically to the detriment of the other. For example, the crop material must be handled, such as by tedding and raking, to help assure the crop material is properly aerated and able to dry thoroughly, however, handling the crop material damages the individual strands and removes leaves such that the nutritional value of the crop material is reduced. The problem with overly handling the crop material is particularly troublesome in instances where the crop material is already dry. As such, the bale creation process must strike a balance between handling the crop material sufficiently to assure the crop material is dry enough to avoid molding while limiting the handling so as not to bring down the crop's nutritional value.

WO 2010/003421 A1 describes a method for optimizing harvesting of crop in which during a production step (mowing, tedding, raking) the moisture of crop deposited on a field is sensed at various locations of the field and provided to a computing system which, also based on a weather forecast, calculates an optimized drying time before the next step of the harvesting process is performed. These production steps are performed on the entire field, even in case that tedding is not required where the material is sufficiently dry.

US 2012/0109614 A1 describes a method for estimating a crop characteristic, in which crop properties on a first field are sensed with a remote (aerial) system. These sensed data and data from other fields, which have already been harvested, are used for estimating the crop characteristic of the first field.

WO 2016/070195 A1 describes a method for estimating the amount of water available to plants based on sensor values from temperature and moisture sensors disposed in an agricultural field.

DE 10 2011 100 054 A1 shows an agricultural machine with a sensor scanning the ground over a width dependent on the width of the implement coupled to the machine.

### Summary

A crop management system for tedding and raking or baling crop material positioned on a field includes a field sensor configured to detect one or more parameters of the crop material at multiple locations in the field and to output a signal representative of the detected parameters at each location, and a data processor in operable communication with the field sensor and configured to receive the signal, and where the data processor is also configured to compile the data representative of the one or more parameters of the crop material at the multiple locations with corresponding position data to determine the time and location of the processing process. The data processor is configured to determine the portions of the field requiring tedding and the portions of the field that do not require tedding.

Data sensors, such as moisture sensors and the like, collect and monitor one or more parameters of a material or location in space. In the present disclosure, one or more moisture sensors are positioned over or moved across a field to detect the moisture content in the cut crop material in a plurality of locations. The sensors then transmit their collected data and corresponding location information to a central data processor to compile and map the data.

Contrary to typical hay creation processes, where entire swaths of field must be tedded, raked, and baled as a single entity based on little more than a farmer's intuition, the data processor of the present disclosure is able to utilize the data provided by the moisture sensors to identify particular locations within the field where the moisture content is too high to be baled and determine the correct time and processes needed to bring the moisture to an acceptable limit. As such, by compiling the data provided by the one or more data sensors, only the crop material in need of tedding and raking is handled. Therefore, relatively dry regions of the field can be left untouched, maximizing their nutritional value, and wet regions can be handled only as necessary until they are dry and ready for baling. Implementations of the disclosure relate to the collection of information regarding the moisture content within the mowed crop material at a particular field location to allow a more accurate determination of where and when the crop material should be tedded, raked, and baled. More specifically, one method includes receiving crop information from one or more field sensors, compiling the crop information and location data to produce a field map, and using the field map to at least partially dictate the parameters of the haymaking process. Furthermore, the field map can be used to determine the most efficient time to rake the crop material into windrows. Still further, the field map can be used to determine the most efficient time to bale the crop material. Still further, the field map can be used to determine the specific locations at which preservatives or other chemicals should be applied.

Other aspects of the disclosure will become apparent by consideration of the detailed description and accompanying drawings.

### BRIEF DESCRIPTION OF THE DRAWINGS

- FIG. 1: is a schematic diagram of a crop management system.
- FIG. 2: is a block diagram of the crop management system of FIG. 1.
- FIG. 3: is a schematic view of a field with a plurality of sensors positioned therein.
- FIG. 4: is a schematic view of a field with a single sensor moving therethrough.
- FIG. 5: illustrates a field map generated by the crop management system of FIG. 1.

### DETAILED DESCRIPTION

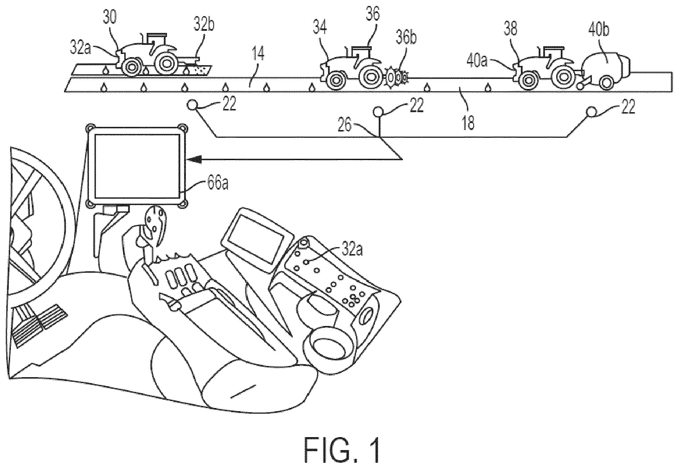

FIG. 1 illustrates a crop management system 10 for detecting and monitoring the moisture content of crop material 14 (i.e., straw, alfalfa, and the like) in a field 18 to aid the haymaking process. More specifically, the resulting haymaking process maximizes the nutrient value within the harvested crop material 14 by minimizing lost leaf matter through less handling of the crop material. Furthermore, the management system 10 provides for a more efficient process by limiting the tedding and chemical application processes to only those regions of the field 18 that require such actions. During use, the crop management system 10 collects data regarding the moisture content in mowed crop material 14 at specific locations in the field 18 and evaluates and/or combines that data to at least partially direct the tedding, raking, and baling processes. For example, the system 10 may utilize the moisture content data to direct, among other things, the timing and location of the tedding process; the timing and location of the raking process; the timing of the baling process; and the timing, location, and quantity of preservatives or other chemicals applied to the crop material.

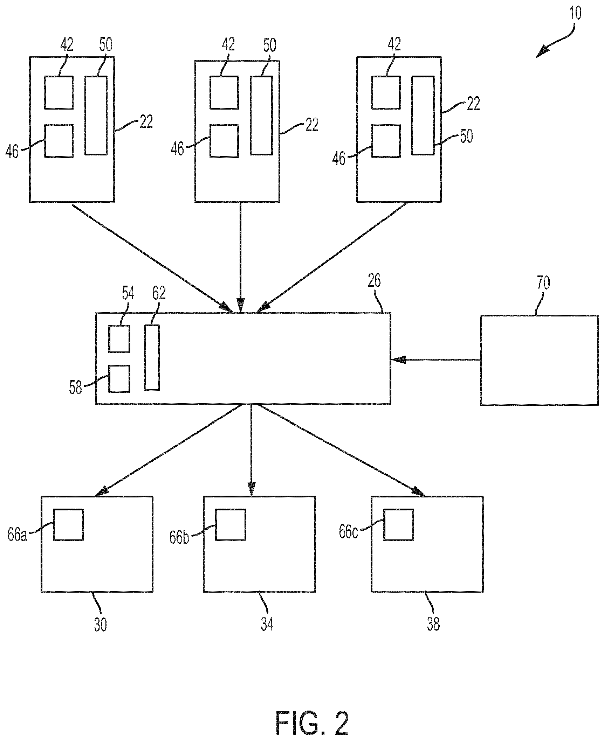

As illustrated in FIGS. 1 and 2, the system 10 includes one or more field sensors 22, a data processor 26 in operable communication with the one or more field sensors 22, and one or more user interfaces 66a, 66b, 66c in operable communication with the data processor 26. The crop management system 10 may also include one or more farm vehicles having tedding, raking, baling, and/or chemical application capabilities. In the illustrated implementation, the crop management system 10 is in operable communication with a tedding tractor 30, a raking tractor 34, and a baling tractor 38. In still other implementations, the crop management system 10 may also be configured for retrofit onto existing farm equipment.

The tedding tractor 30 of the present implementation includes a first drive unit or tractor 32a with a tedding attachment 32b coupled thereto. During use, the tedding attachment 32b uses a plurality of moving forks to aerate or "wuffle" the hay and speed up the process of haymaking by allowing the hay to dry or cure more evenly and quickly.

The raking tractor 34 of the present implementation includes a second drive unit or tractor 36a with a raking attachment 36b coupled thereto. During use, the raking attachment 36b collects the mowed crop material and combines it into windrows for subsequent collection. The raking attachment 36b may also fluff up the hay and turn it over to aid the drying process.

The baling tractor 38 of the present implementation includes a third drive unit or tractor 40a with a baling attachment 40b coupled thereto. During use, the baling attachment 40b collects the crop material 14 in the windrows and compacts the material 14 into individual bales for subsequent use.

Although not illustrated, the crop management system 10 may also include a chemical tractor (not shown) having a chemical attachment or trailer for applying pesticides, drying agents, fertilizer, and the like to the crop material 14.

While the present disclosure describes each of the three tractors 30, 34, 38 as separate items, it is to be understood some tractors may be coupled to multiple attachments and used for more than one process. Furthermore, the three tractors 30, 34, 38 may be used simultaneously or separately, and at different times, during the haymaking process.

Illustrated in FIGS. 1-3, each field sensor 22 of the crop management system 10 is in operable communication with the data processor 26 and is configured to detect one or more agricultural field parameters from the mowed crop material 14 in its general vicinity. In the illustrated implementation, each field sensor 22 includes a moisture sensor 42 able to determine the moisture level within the crop material 14 at a given location. Each field sensor 22 may also include a location device or GPS 46 to determine the location of the sensor 22 with respect to the field 18 and a transmitter 50 to communicate the moisture and position data to the data processor 26 during use.

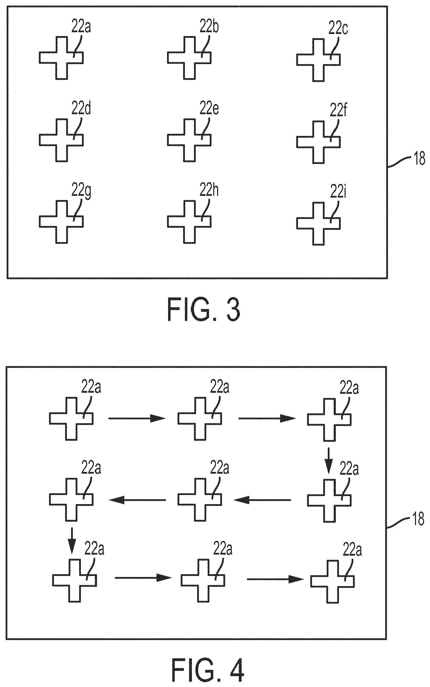

Illustrated in FIG. 3, a first implementation of the crop management system 10 includes a plurality of fixed field sensors 22a-i, each positioned evenly throughout the field 18 in a substantially rectangular array. In such an implementation, the plurality of sensors 22a-i remain in a fixed position relative to the field which allows each individual sensor 22a-i to continuously detect the moisture level at its particular location. Due to the stationary nature of the sensors 22a-i, the sensors may not need a location device 46 if the location of the sensor 22a-i is predetermined. While the sensors 22a-i of the illustrated implementation are positioned in a rectangular array; in alternative implementations, the sensors 22a-i may be distributed over the field 18 in any pattern that provides sufficient coverage including a spiral pattern, and the like.

Although not illustrated the fixed field sensors 22a-i may be positioned throughout the field in a manner similar to survey markers. In other implementations, the sensors 22a-i may be buried underground at various locations throughout the field. In other implementations, the field sensors 22a-i may be scattered over the field. In still other implementations, the sensors 22a-i may be biodegradable. Still further, in some implementations, the sensors 22a-i may be positioned over the entire field, while in other implementations, the sensors 22a-i may only be positioned in certain sub-sections or locations of the field.

Illustrated in FIG. 4, a second implementation of the crop management system 10 includes one or more field sensors 22a that move with respect to the field 18. In such an implementation, the single sensor 22a may cover the entire field 18 but may only provide information regarding a single location at any one time. In such implementations, the field sensor 22a may be coupled to and move with a tractor (not shown) taking successive readings of the moisture level of the crop material 14 shortly after it has been mowed. In still other implementations, the field sensor 22a may be coupled to a drone or other moveable device (not shown) to move independently of any of the tractors 30, 34, 38. In such implementations, the sensor 22a may move in a predetermined pattern (such as a spiral, back and forth, and the like) to provide even coverage over the entire field 18 (see Fig. 4), or alternatively, the sensor 22a may be directed to travel toward or around a specific location on the field 18 to provide more focused coverage.

Although not illustrated, in another implementation of the crop management system 10 a combination of both stationary sensors and movable sensors may be used. In such implementations, the stationary sensors may provide a data regarding the entire field while the movable sensors supplement that data as it moves across the field. As such, the data processor 26 would take into account both data sets when determining the specifics of the baling process.

The data processor 26 of the management system 10 includes a central processing unit or CPU 54, a memory unit 58 in operable communication with the CPU 54, and a communication module 62 in operable communication with the CPU 54. In the illustrated implementation, the data processor 26 is in operable communication with the one or more field sensors 22 via the communication module 62. The data processor 26 is also in operable communication with a plurality of remote user interfaces 66a, 66b, 66c, each of which may be associated with a corresponding tractor 30, 34, 38. In the illustrated implementation, the communication module 62 is a wireless system using Bluetooth, WiFi, or other similar technologies. However, in alternative implementations, other types of communication modules, including wired, may be used. In still other implementations, the user interfaces 66a, 66b, 66c may be stand-alone items that can be carried or temporarily installed in one of the tractors during use.

The data processor 26 is also in operable communication with a weather input 70 able to provide up-to-date weather forecasts of weather conditions at and around the field 18. The weather input 70 may be a signal provided by a remote source (i.e., the internet, the national weather service, and the like) or the weather input 70 may be local, i.e., a dedicated station built on site (not shown).

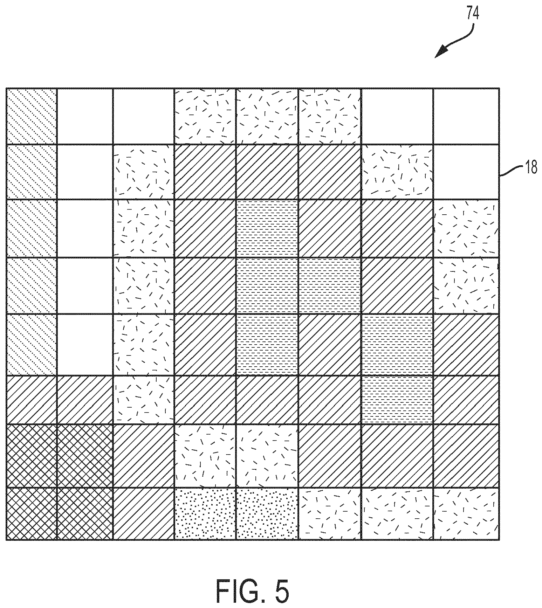

During operation of the management system 10, the CPU 54 continuously receives data from each field sensor 22 in the form of moisture level and position data. The CPU 54 then compiles the moisture level with its associated position data to create data points on a field map 74, or another form of 2-D representation of the moisture levels at various locations over the entire field 18 (see Fig. 5). Depending upon the number of sensors 22 and the manner in which the data is collected (e.g., the first or second implementations of the field sensors 22, described above), the resolution of the resulting field map 74 may vary. Still further, the CPU 54 may include software or other algorithms that allow the CPU 54 to estimate the moisture levels between data points provided by the field sensors 22, allowing for a more continuous map.

The CPU 54 is also configured to apply the compiled data to one or more algorithms and provide outputs to the remote user interfaces 66a, 66b, 66c, generally in the form of instructions to a user or operator regarding the parameters of the tedding, raking, and baling processes (described below). In some implementations, the CPU 54 provides information to the user in the form of maps, textual instructions, verbal instructions, graphical displays, operation settings and the like that allow the user to drive or otherwise operate the necessary equipment (i.e., the tractors 30, 34, 38) in the desired manner. For example, the CPU 54 may provide the remote user interface 66a of the tedding tractor 30 with a graphical map indicating the location(s) of the field 18 that require tedding. In other examples, the CPU 54 may provide the remote user interface 66a of the tedding tractor 30 with verbal or visual driving instructions or coordinates. The CPU 54 may also provide the remote user interface 66a with instructions regarding when to engage or disengage the tedding mechanism 32b or at what settings to operate the tedding mechanism 32b. In still other implementations, the CPU 54 may directly control the tractors 30, 34, 38 during the haymaking process. In still other implementations, the individual user interfaces 66a, 66b, 66c may include their own GPS or positioning device (not shown) to allow turn-by-turn navigation instructions or to display the relative positions of the tractor and the location in need of attention.

The CPU 54 uses the moisture data from the sensors 22 to calculate the parameters of the tedding process. When doing so, the CPU 54 reviews the resulting moisture and location data to determine what, if any, locations require additional assistance to dry. After doing so, the CPU 54 calculates which locations of the field require tedding and which locations of the field do not require tedding. In some implementations, the CPU 54 compares the moisture data to a predetermined maximum moisture threshold. In instances where the moisture level in one or more particular locations exceeds the maximum moisture threshold, the CPU 54 marks that location for tedding and indicates that information to the user via the remote user interface 66a. In other implementations, the CPU 54 may compare the moisture data to an acceptable envelope of moisture levels and time tables. In still other implementations, the CPU 54 may take into account the terrain, weather, crop type, and the like to determine if a particular location or area is in need of tedding.

Once all the locations in need of tedding are identified, the CPU 54 may also calculate the most fuel efficient path between the locations that require tedding to help save running time and fuel costs. In still other implementations, the CPU 54 may also use current moisture readings and predictive models to calculate the optimal time at which to begin the tedding process (described below). When doing so, the CPU 54 may calculate a start time taking into account all locations that require tedding; however in alternative implementations, the CPU 54 may calculate a unique start time for each individual location that requires tedding. In still other implementations, the CPU 54 may use weather data to predict the latest time one can ted the field before encountering rain or other inclement weather.

The CPU 54 also uses the moisture data from the sensors 22 to calculate the parameters of the raking process and the baling processes. When doing so, the CPU 54 reviews the resulting moisture and location data to predict which locations of the field require raking and which locations of the field do not require raking. Furthermore, the CPU 54 calculates what time(s) the raking and baling process should begin. To do this, the CPU 54 compares the current and prior moisture and location data in the memory unit 58 to a predetermined drying rate algorithm to produce a predictive drying model. When creating the predictive drying model, the CPU 54 may take into account, among other things, the grade of the land on which the crop 14 is positioned, the type of crop 14 being harvested, and the current weather conditions via the weather input 70. The CPU 54 then applies the predictive drying model to the current moisture conditions, using them as a starting point to predict the times at which the moisture level within the crop material 14 will be ideal for both the raking and baling process. The ideal time for both processes is then communicated to the user via the remote user interfaces 66b, 66c. In instances where the moisture level in the field 18 is uneven, the CPU 54 may also develop a specific path or multiple start times to take into account any conflicting data. More specifically, the CPU 54 may divide the field into multiple sub-units, and calculate a unique start time for each sub-unit. When dividing the field into the sub-units, the CPU 54 may take into account numerous factors, such as but not limited to, similar soil type, similar grade, similar shade or sun exposure, similar terrain features, and the like.

The CPU 54 further uses the moisture data from the sensors 22 to calculate the parameters of the chemical application process. When doing so, the CPU 54 reviews the resulting moisture and location data to determine what, if any, locations require preservatives, drying agents, fertilizers, or other chemical additives. In instances where the moisture level is deemed appropriate for chemical additives, the CPU 54 marks that location for spraying and indicates that information to the user via the remote user interface 66c in the baling tractor 38. Once all the locations in need of chemical application have been identified, the CPU 54 may also calculate the most fuel efficient path between each of the locations to help save running time and fuel costs. In still other implementations, the CPU 54 may also calculate the optimal wait time before beginning the chemical application process. In still other implementations, the CPU 54 may use the moisture or other crop related data from the sensors 22 to determine the quantity of chemicals that need to be applied, or the specific type of chemical that needs to be applied.

In still other implementations, the CPU 54 may use data from some processes to effect or update the calculation of subsequent processes. For example, the CPU may use moisture data from the sensors 22 to calculate the ideal tedding process, and then use the results of the tedding process to update the parameters of the ideal raking and baling process. In still other implementations, the CPU 54 may combine instructions when sending information to a tractor 38 having multiple capabilities. For example, the CPU 54 may combine and optimize the ideal tedding and raking processes and provide the results to a tractor having both tedding and raking capabilities.

In still other implementations, various tractors may directly communicate with one another to create a network of moving sensors 22a positioned at different locations on the field. In such implementations, each moving sensor 22a can supplement the data provided by other sensors 22a to create a single, interconnected field map 74. In still other implementations, the various sensors (either fixed or moving) may be in communication with a separate computer (e.g., an office computer) or online to allow a third party to monitor the progress of the baling operation.

The CPU may also provide all of the aforementioned information to a remote location for additional tracking, monitoring, or storing, and for real-time or future applications.

## Claims

1. A crop management system (10) for processing crop material (14) positioned on a field (18), the crop management system (10) comprising:
a field sensor (22) configured to detect one or more parameters of the crop material (14) at multiple locations in the field (18) and to output a signal representative of the detected parameters at each location; and
a data processor (26) in operable communication with the field sensor (22) and configured to receive the signal, and wherein the data processor (26) is also configured to compile the data representative of the one or more parameters of the crop material (14) at the multiple locations with corresponding position data to determine the timing and location of a processing process comprising tedding crop material,
**characterized in that** the data processor (26) is configured to determine the portions of the field (18) requiring tedding and the portions of the field (18) that do not require tedding.

2. The crop management system (10) of claim 1, wherein the data processor (26) is configured to combine the data representative of the one or more parameters of the crop material (14) at the multiple locations and corresponding position data to produce data points on a field map.

3. The crop management system (10) of claim 1 or 2, wherein the data processor (26) is configured to combine the data representative of the one or more parameters of the crop material (14) at the multiple locations with corresponding position data to produce data points on a field map, and wherein the field map is displayed on a user interface.

4. The crop management system (10) of one of the claims 1 to 3, wherein the field sensor (22) is a moisture sensor able to determine the moisture level within the crop material (14) at each location.

5. The crop management system (10) of one of the claims 1 to 4, wherein the field sensor (22) is movable with respect to the field (18) between a first location and a second location different than the first location.

6. The crop management system (10) of one of the claims 1 to 5, wherein the data processor (26) is configured to determine the time at which tedding in a particular location should begin.

7. The crop management system (10) of one of the claims 1 to 6, wherein the field sensor (22) is mounted on a first vehicle, and wherein the tedding is conducted by a second vehicle different than the first vehicle.

8. The crop management system (10) of one of the claims 1 to 5, wherein the processing process comprises raking crop material (14) and wherein the field sensor (22) is positioned on a first vehicle, and wherein the raking process is conducted by a second vehicle different than the first vehicle.

9. The crop management system (10) of one of the claims 1 to 5 or 8, wherein the processing process comprises raking crop material (14) and wherein the data processor (26) is configured to determine locations on the field (18) where raking is required, and locations on the field (18) where raking is not required.

10. The crop management system (10) of one of the claims 1 to 5, 8 or 9, wherein the processing process comprises raking crop material (14) and wherein the data processor (26) is configured to determine the timing at which raking should occur at each location on the field (18) where raking is required.

11. The crop management system (10) of one of the claims 1 to 5, wherein the processing process comprises baling crop material (14) and wherein the field sensor (22) is positioned on vehicle first vehicle, and wherein the baling process is conducted by a second vehicle different than the first vehicle.

12. The crop management system (10) of one of the claims 1 to 5 or 11, wherein the processing process comprises baling crop material (14) and wherein the data processor (26) is configured to determine locations on the field (18) where baling is required, and locations on the field (18) where baling is not required.

13. The crop management system (10) of one of the claims 1 to 5, 11 or 12, wherein the processing process comprises baling crop material (14) and wherein the data processor (26) is configured to determine the time at which baling should occur at each location on the field (18) where baling is required.

14. The crop management system (10) of one of the claims 1 to 5, wherein the processing process comprises applying chemicals to crop material (14) and wherein the data processor (26) is configured to determine the quantity of chemical that needs to be applied.

## Patentansprüche

1. Erntegutmanagementsystem (10) zum Verarbeiten von Erntegutmaterial (14), das auf einem Feld (18) positioniert ist, wobei das Erntegutmanagementsystem (10) Folgendes umfasst:
einen Feldsensor (22), der konfiguriert ist, einen oder mehrere Parameter des Erntegutmaterials (14) an mehreren Orten auf dem Feld (18) zu detektieren, und ein Signal, das die am jedem Ort detektierten Parameter darstellt, auszugeben; und
einen Datenprozessor (26), der in Kommunikation mit dem Feldsensor (22) betrieben werden kann, und konfiguriert ist, das Signal zu empfangen, und wobei der Datenprozessor (26) auch konfiguriert ist, die Daten, die den einen oder die mehreren Parameter des Erntegutmaterials (14) an den mehreren Orten darstellen, mit entsprechenden Positionsdaten zu kombinieren, um den Zeitpunkt und den Ort eines Verarbeitungsprozesses, der das Ausbreiten zum Trocken von Erntegutmaterial umfasst, zu bestimmen,
**dadurch gekennzeichnet, dass** der Datenprozessor (26) konfiguriert ist, die Abschnitte auf dem Feld (18), die das Ausbreiten zum Trocken erfordern, und die Abschnitte auf dem Feld (18), die das Ausbreiten zum Trocken nicht erfordern, zu bestimmen.

2. Erntegutmanagementsystem (10) nach Anspruch 1, wobei der Datenprozessor (26) konfiguriert ist, die Daten, die den einen oder die mehreren Parameter des Erntegutmaterials (14) an den mehreren Orten darstellen, und entsprechende Positionsdaten zu kombinieren, um Datenpunkte auf einer Karte des Felds zu erzeugen.

3. Erntegutmanagementsystem (10) nach Anspruch 1 oder 2, wobei der Datenprozessor (26) konfiguriert ist, die Daten, die den einen oder die mehreren Parameter des Erntegutmaterials (14) an den mehreren orten darstellen, mit den entsprechenden Positionsdaten zu kombinieren, um Datenpunkte auf einer Karte des Felds zu erzeugen, und wobei die Karte des Feldes auf einer Benutzerschnittstelle angezeigt wird.

4. Erntegutmanagementsystem (10) nach einem der Ansprüche 1 bis 3, wobei der Feldsensor (22) einem Feuchtigkeitssensor entspricht, der den Feuchtigkeitsgehalt in dem Erntegutmaterial (14) an jedem Ort bestimmen kann.

5. Erntegutmanagementsystem (10) nach einem der Ansprüche 1 bis 4, wobei der Feldsensor (22) bezüglich des Feldes (18) zwischen einem ersten Ort und einem zweiten Ort, der sich von dem ersten Ort unterscheidet, bewegt werden kann.

6. Erntegutmanagementsystem (10) nach einem der Ansprüche 1 bis 5, wobei der Datenprozessor (26) konfiguriert ist, den Zeitpunkt zu bestimmen, an dem das Ausbreiten zum Trocken an einem bestimmten Ort beginnen soll.

7. Erntegutmanagementsystem (10) nach einem der Ansprüche 1 bis 6, wobei der Feldsensor (22) an einem ersten Fahrzeug montiert ist, und wobei das Ausbreiten zum Trocken von einem zweiten Fahrzeug, das sich von dem ersten Fahrzeug unterscheidet, durchgeführt wird.

8. Erntegutmanagementsystem (10) nach einem der Ansprüche 1 bis 5, wobei der Verarbeitungsprozess das Harken von Erntegutmaterial (14) umfasst, und wobei der Feldsensor (22) auf einem ersten Fahrzeug positioniert ist, und wobei das Harkprozess von einem zweiten Fahrzeug, das sich von dem ersten Fahrzeug unterscheidet, durchgeführt wird.

9. Erntegutmanagementsystem (10) nach einem der Ansprüche 1 bis 5 oder 8, wobei der Verarbeitungsprozess das Harken von Erntegutmaterial (14) umfasst, und wobei der Datenprozessor (26) konfiguriert ist, Orte auf dem Feld (18), an denen Harken erforderlich ist, und Orte auf dem Feld (18), an denen Harken nicht erforderlich ist, zu bestimmen.

10. Erntegutmanagementsystem (10) nach einem der Ansprüche 1 bis 5, 8 oder 9, wobei der Verarbeitungsprozess das Harken von Erntegutmaterial (14) umfasst, und wobei der Datenprozessor (26) konfiguriert ist, den Zeitpunkt zu bestimmen, an dem Harken an jedem Ort auf dem Feld (18), an dem Harken erforderlich ist, geschehen soll.

11. Erntegutmanagementsystem (10) nach einem der Ansprüche 1 bis 5, wobei der Verarbeitungsprozess das in Ballen Verpacken von Erntegutmaterial (14) umfasst, und wobei der Feldsensor (22) auf dem ersten Fahrzeug positioniert ist, und wobei der Prozess des in Ballen Verpackens von einen zweiten Fahrzeug, das sich von dem ersten Fahrzeug unterscheidet, durchgeführt wird.

12. Erntegutmanagementsystem (10) nach einem der Ansprüche 1 bis 5 oder 11, wobei der verarbeitungsprozess das in Ballen verpacken von Erntegutmaterial (14) umfasst, und wobei der Datenprozessor (26) konfiguriert ist, Orte auf dem Feld (18), an denen in Ballen Verpacken erforderlich ist, und Orte auf dem Feld (18), an denen in Ballen Verpacken nicht erforderlich ist, zu bestimmen.

13. Erntegutmanagementsystem (10) nach einem der Ansprüche 1 bis 5, 11 oder 12, wobei der Verarbeitungsprozess das in Ballen Verpacken von Erntegutmaterial (14) umfasst, und wobei der Datenprozessor (26) konfiguriert ist, den Zeitpunkt zu bestimmen, an dem das in Ballen Verpacken an jedem Ort auf dem Feld (18), an dem in Ballen Verpacken erforderlich ist, geschehen soll.

14. Erntegutmanagementsystem (10) nach einem der Ansprüche 1 bis 5, wobei der Verarbeitungsprozess das Anwenden von Chemikalien auf das Erntegutmaterial (14) umfasst, und wobei der Datenprozessor (26) konfiguriert ist, die Menge der Chemikalie, die angewendet werden muss, zu bestimmen.

## Revendications

1. Système (10) de gestion de récolte pour le traitement d'un produit de récolte (14) situé sur un champ (18), le système (10) de gestion de récolte comprenant :
un capteur de champ (22) configuré pour détecter un ou plusieurs paramètres du produit de récolte (14) à de multiples emplacements dans le champ (18) et délivrer un signal représentant les paramètres détectés à chaque emplacement ; et
un processeur de données (26) en communication fonctionnelle avec le capteur de champ (22) et configuré pour recevoir le signal, et le processeur de données (26) étant également configuré pour compiler les données représentant les un ou plusieurs paramètres du produit de récolte (14) aux multiples emplacements avec des données de position correspondantes afin de déterminer le moment opportun et l'emplacement d'un processus de traitement comprenant un fanage du produit de récolte,
**caractérisé en ce que** le processeur de données (26) est configuré pour déterminer les parties du champ (18) nécessitant un fanage et les parties du champ (18) qui ne nécessitent pas un fanage.

2. Système (10) de gestion de récolte selon la revendication 1, dans lequel le processeur de données (26) est configuré pour combiner les données représentant les un ou plusieurs paramètres du produit de récolte (14) aux multiples emplacements et des données de position correspondantes afin de produire des points de données sur un plan du champ.

3. Système (10) de gestion de récolte selon la revendication 1 ou 2, dans lequel le processeur de données (26) est configuré pour combiner les données représentant les un ou plusieurs paramètres du produit de récolte (14) aux multiples emplacements à des données de position correspondantes afin de produire des points de données sur un plan du champ, et dans lequel le plan du champ est affiché sur une interface-utilisateur.

4. Système (10) de gestion de récolte selon l'une des revendications 1 à 3, dans lequel le capteur de champ (22) est un capteur d'humidité apte à déterminer le niveau d'humidité au sein du produit de récolte (14) à chaque emplacement.

5. Système (10) de gestion de récolte selon l'une des revendications 1 à 4, dans lequel le capteur de champ (22) est mobile par rapport au champ (18) entre un premier emplacement et un deuxième emplacement différent du premier emplacement.

6. système (10) de gestion de récolte selon l'une des revendications 1 à 5, dans lequel le processeur de données (26) est configuré pour déterminer le moment auquel un fanage à un emplacement particulier doit débuter.

7. Système (10) de gestion de récolte selon l'une des revendications 1 à 6, dans lequel le capteur de champ (22) est monté sur un premier véhicule, et dans lequel le fanage est effectué par un deuxième véhicule différent du premier véhicule.

8. Système (10) de gestion de récolte selon l'une des revendications 1 à 5, dans lequel le processus de traitement comprend un andainage du produit de récolte (14), et dans lequel le capteur de champ (22) est situé sur un premier véhicule, et dans lequel le processus d'andainage est effectué par un deuxième véhicule différent du premier véhicule.

9. Système (10) de gestion de récolte selon l'une des revendications 1 à 5 ou 8, dans lequel le processus de traitement comprend un andainage du produit de récolte (14), et dans lequel le processeur de données (26) est configuré pour déterminer des emplacements sur le champ (18) où un andainage est nécessaire et des emplacements sur le champ (18) où un andainage n'est pas nécessaire.

10. Système (10) de gestion de récolte selon l'une des revendications 1 à 5, 8 ou 9, dans lequel le processus de traitement comprend un andainage du produit de récolte (14), et dans lequel le processeur de données (26) est configuré pour déterminer le moment opportun auquel un andainage doit avoir lieu à chaque emplacement sur le champ (18) où un andainage est nécessaire.

11. Système (10) de gestion de récolte selon l'une des revendications 1 à 5, dans lequel le processus de traitement comprend une mise en balles du produit de récolte (14), et dans lequel le capteur de champ (22) est situé sur un premier véhicule, et dans lequel le processus de mise en balles est effectué par un deuxième véhicule différent du premier véhicule.

12. Système (10) de gestion de récolte selon l'une des revendications 1 à 5 ou 11, dans lequel le processus de traitement comprend une mise en balles du produit de récolte (14), et dans lequel le processeur de données (26) est configuré pour déterminer des emplacements sur le champ (18) où une mise en balles est nécessaire et des emplacements sur le champ (18) où une mise en balles n'est pas nécessaire.

13. Système (10) de gestion de récolte selon l'une des revendications 1 à 5, 11 ou 12, dans lequel le processus de traitement comprend une mise en balles du produit de récolte (14), et dans lequel le processeur de données (26) est configuré pour déterminer le moment auquel une mise en balles doit avoir lieu à chaque emplacement sur le champ (18) où une mise en balles est nécessaire.

14. Système (10) de gestion de récolte selon l'une des revendications 1 à 5, dans lequel le processus de traitement comprend l'application de produits chimiques sur le produit de récolte (14), et dans lequel le processeur de données (26) est configuré pour déterminer la quantité de produit chimique devant être appliquée.
